(19) Europäisches Patentamt
European Patent Office
Office européen des brevets

(11) EP 2 324 819 B1

(12) # FASCICULE DE BREVET EUROPEEN

(45) Date de publication et mention de la délivrance du brevet:
**08.07.2015 Bulletin 2015/28**

(51) Int Cl.:
*A61Q 13/00* (2006.01) *A61K 8/58* (2006.01)

(21) Numéro de dépôt: **10189860.9**

(22) Date de dépôt: **03.11.2010**

(54) **Composition parfumante colorée sans diphénylacrylate d'alkyle contenant un filtre uva hydrophile**

Gefärbte Parfum-Zusammensetzung enthaltend einen hydrophilen UVA-Filter ohne Alkyl Diphenylacrylat

Coloured perfume composition comprising a hydrophilic UVA filter without alkyl diphenylacrylate

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **19.11.2009 FR 0958181**
**19.11.2009 FR 0958183**

(43) Date de publication de la demande:
**25.05.2011 Bulletin 2011/21**

(73) Titulaire: **L'Oréal**
**75008 Paris (FR)**

(72) Inventeurs:
- **Thfoin, Willy**
  **91200, Massy (FR)**
- **Grimal, Boris**
  **94260, Fresnes (FR)**

(74) Mandataire: **Kromer, Christophe**
**L'Oréal**
**D.I.P.I.**
**25-29 Quai Aulagnier**
**92665 Asnières-sur-Seine (FR)**

(56) Documents cités:
**EP-A1- 1 897 592 WO-A1-00/25730**

- **ANONYMOUS: "Ingredient Protection - UV Light Stabilisers", , 2007, pages 1-36, XP002591927, Extrait de l'Internet: URL:https:// 128.246.4.236/pf/pf_docMDMS.as p? itemnumber=8806567&docnumber=24470&app_ i d=ACROBAT&targetlibrary=_ SalesAndMarketing &dt=PRES&ind= [extrait le 2010-07-14]**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 324 819 B1

## Description

L'invention se rapporte à une composition parfumante colorée comprenant dans un milieu cosmétiquement acceptable :

a) au moins 2% en poids d'une substance parfumante par rapport au poids total de la composition ;
b) au moins une silicone benzotriazole de formule (1) particulière que l'on définira plus loin en détail ;
c) au moins un colorant soluble dans ledit milieu ; ladite composition ne contenant pas de composé β,β'-diphénylacrylate d'alkyle ni de composé α-cyano-β,β'-diphénylacrylate d'alkyle.

On sait qu'un parfum est l'association de différentes substances odorantes qui s'évaporent à des périodes différentes. Chaque parfum présente ce que l'on appelle une « note de tète » qui est l'odeur diffusant en premier lors de l'application du parfum ou lors de l'ouverture du récipient le contenant, une « note de coeur ou corps » qui correspond au parfum complet (émission pendant quelques heures après la « note de tête ») et une « note de fond » qui est l'odeur la plus persistante (émission pendant plusieurs heures après la « note de coeur »). La persistance de la note de coeur et de la note de fond correspond à la rémanence du parfum.

L'être humain a de tout temps cherché à se parfumer et à parfumer les objets qui l'entourent ou les lieux dans lesquels il se trouve, et ceci, aussi bien pour masquer des odeurs fortes et/ou désagréables que pour donner une bonne odeur.

Il est courant d'incorporer du parfum dans un certain nombre de produits ou compositions, en particulier cosmétiques et dermatologiques telles que des eaux fraîches, eaux de toilette, des eaux de parfum, des lotions après rasage, des eaux de soin. Pour des raisons esthétiques et de coût de fabrication, on colore le jus des parfums en ajoutant une quantité efficace de colorant soluble dans le support de la formulation (généralement alcoolique ou hydroalcoolique) plutôt que de teindre ou laquer le flacon qui est une opération industrielle plus onéreuse. La couleur mise au point dans ces formulations parfumées doit rester stable aussi bien dans le temps, qu'exposée à la lumière. On ajoute généralement un système filtrant et/ou un système antioxydant.

On a proposé dans la demande EP1897592 des compositions parfumantes colorées comprenant comme sytème stabilisant au moins 0,2% en poids par rapport au poids total de la composition d'au moins un composé β,β'-diphénylacrylate d'alkyle ou α-cyano-β,β'-diphénylacrylate d'alkyle comme l'octocrylène par rapport au poids total de la composition et au moins un filtre organique UV-A soluble dans ledit milieu

Cependant l'utilisation d'un composé β,β'-diphénylacrylate d'alkyle ou d'un composé α-cyano-β,β'-diphénylacrylate d'alkyle comme l'octocrylène et d'un filtre UVA avait tendance d'un part à produire des recristallisations imprévisibles selon le concentré de parfum utilisé. D'autre part, la demanderesse a découvert au cours de ses recherches qu'un composé β,β'-diphénylacrylate d'alkyle ou un composé α-cyano-β,β'-diphénylacrylate d'alkyle comme l'octocrylène inhibait la stabilisation de la couleur notamment dans les domaines des jaunes et celui des violets.

On a proposé dans la demande WO2005/042828 des compositions parfumantes colorées comprenant comme système stabilisant un dérivé de pipéridinol (ie Tris(tétraméthylhydroxypipéridinol) citrate - Tinoguard Q) associé à un filtre UV organique choisi parmi les dérivés de dibenzoylméthane, les cinnamates, les dérivés de camphres et le s-triazines. L'exemple 6 décrit notamment une eau de toilette comprenant un parfum, un colorant, 0,1% d'un mélange de Butylmethoxydibenzoylmethane et d'octocrylene, un stabilisant du type pipéridinol

Cependant, ces dérivés de pipéridinol présentent l'inconvénient de générer un jaunissement des eaux de toilettes ainsi qu'une odeur parasite.

On a préconisé également dans la demande WO00/25370 des eaux de toilettes colorées stabilisées par un composé benzotriazole particulier et/ou une triazine particulière comme par exemple le Sodium Benzotriazolyl Butylphenol Sulfonate comme le produit vendu sous le nom « TINOGUARD HS » par la société CIBA-GEIGY ; le Benzotriazolyl dodécyl p-Cresol comme le produit vendu sous le nom « TINOGUARD TL » par la société CIBA-GEIGY. comme le produit vendu sous le nom commercial « CIBAFAST H Liquid » par la société CIBA-GEIGY, Bumetrizole comme le produit vendu sous le nom « TINOGUARD AS » par la société CIBA-GEIGY. Dans les demandes WO09/059872 et FR2923386, on a proposé l'utilisation d'un composé benzotriazole particulier notamment le Bumetrizole (TINOGUARD AS) en association avec d'autres filtres particuliers tel qu'un dérivé de dibenzoylméthane avec un un composé β ,β'-diphénylacrylate d'alkyle ou α-cyano-β,β'-diphénylacrylate d'alkyle

Cependant, ces composés de benzotriazole en particulier Bumetrizole (TINOGUARD AS) sont difficilement solubles dans les eaux de toilettes, ont tendance à recristalliser dans le temps, en plus d'avoir d'un pouvoir stabilisant médiocre.

On a proposé aussi dans les demandes de brevet EP1994921, FR 2916347, FR 2916348, et FR 2916349 l'utilisation d'un filtre UV du type aminobenzophénone comme le 2-(4-diethylamino-2-hydroxybenzoyl)-benzoate de n-hexyle vendu sous le nom commercial« UVINUL A +» avec d'autres filtres particuliers afin de stabiliser la couleur des eaux de toilettes. Cependant, les filtres aminobenzophénone comme l'Uvinul A+ apporte une couleur jaune qui n'est

pas souhaitable pour les formulations parfumées de couleur pâle différent du jaune.

Il subsiste le besoin de produits parfumants colorés ne présentant pas les inconvénients des produits de l'art antérieur, et notamment le besoin de produits parfumants colorés dont la couleur reste stable dans le temps et sous les effets de la lumière.

La demanderesse a découvert de manière surprenante que cet objectif pouvait être atteint en utilisant une composition parfumante colorée comprenant dans un milieu cosmétiquement acceptable :

a) au moins 2% en poids d'une substance parfumante par rapport au poids total de la composition ;
b) au moins une silicone benzotriazole de formule (1) particulière que l'on définira plus loin en détail ;
c) au moins un colorant soluble dans ledit milieu ; ladite composition ne contenant pas de composé β,β'-diphénylacrylate d'alkyle ni de composé α -cyano-β,β'-diphénylacrylate d'alkyle.

Cette découverte est à la base de l'invention.

L'invention a encore pour objet une composition parfumante colorée comprenant dans un milieu cosmétiquement acceptable :

a) au moins 2% en poids d'une substance parfumante par rapport au poids total de la composition ;
b) au moins une silicone benzotriazole de formule (1) particulière que l'on définira plus loin en détail ;
c) au moins un colorant soluble dans ledit milieu ; ladite composition ne contenant pas de composé β,β'-diphénylacrylate d'alkyle ni de composé α -cyano-β,β'-diphénylacrylate d'alkyle.

L'invention a encore pour objet un procédé de parfumage des matières kératiniques humaines et notamment de la peau, des lèvres, des cheveux, du cuir chevelu, les cils et sourcils, les ongles ou d'un vêtement comprenant l'application sur les matières kératiniques ou ledit vêtement de la composition telle que définie ci-dessus.

L'invention concerne également l'utilisation d'au moins une silicone benzotriazole de formule (I), comme agent stabilisant des propriétés organoleptiques d'une composition cosmétique parfumante colorée vis-à-vis des agressions extérieures notamment de la lumière ou des différences de température en particulier la couleur et/ou l'odeur de ladite composition ; ladite composition ne contenant pas de composé β ,β'-diphénylacrylate d'alkyle ni de composé α-cyano-β,β'-diphénylacrylate d'alkyle.

Par composition parfumante, on entend toute composition laissant après application sur les matières kétatiniques un parfum.

Par « substance parfumante», on entend tout parfum ou arôme susceptible de parfumer la peau et les matières kératiniques humaines en général comprenant la peau, les cheveux, le cuir chevelu, les lèvres ; les ongles.

On entend par « matières kératiniques humaines », la peau (visage, corps, les lèvres, intérieur des paupières), le cuir chevelu, les cheveux, les cils, les sourcils, les ongles, les muqueuses.

On entend par « milieu cosmétiquement acceptable » dans la composition de l'invention, un milieu non toxique et susceptible d'être appliqué sur les matières kératiniques humaines comprenant la peau, les lèvres, les ongles , les cheveux, le cuir chevelu, les cils, les sourcils.

On entend par ne contenant pas de composé β,β'-diphénylacrylate d'alkyle ou de composé α-cyano-β,β'-diphénylacrylate d'alkyle comme contenant moins de 0,2% en poids de composé β,β'-diphénylacrylate d'alkyle ou α -cyano-β,β'-diphénylacrylate d'alkyle voire moins de 0,1% en poids voire exempte de composé β,β'-diphénylacrylate d'alkyle ni de composé α -cyano-β,β'-diphénylacrylate d'alkyle.

## DERIVES SILICIES DE BENZOTRIAZOLE

Les dérivés siliciés à fonction benzotriazole utilisés dans la présente invention sont de préférence des silanes ou des siloxanes à fonction benzotriazole comprenant au moins une unité de formule (1) suivante :

$$O_{(3-a)/2}Si(R)_a\text{-}G \qquad (1)$$

- R représente un radical alkyle en $C_1$-$C_{10}$ éventuellement halogéné ou un radical phényle ou un radical triméthylsilyloxy,
- a est un nombre entier choisi entre 0 et 3 inclusivement,
- et le symbole G désigne un radical monovalent lié directement à un atome de silicium, et qui répond à la formule (2) suivante :

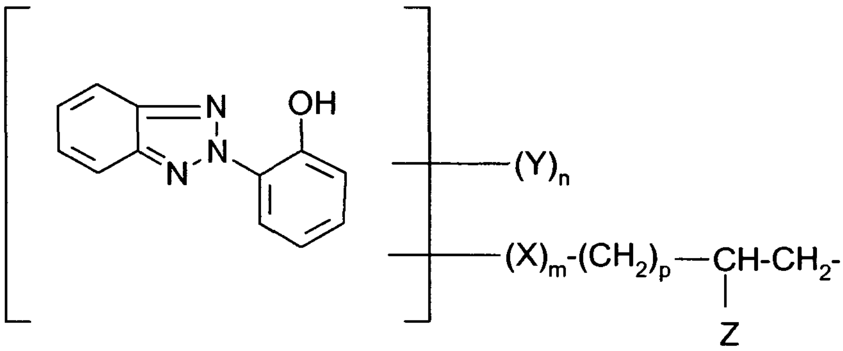

(2)

dans laquelle :

- Y, identiques ou différents, sont choisis parmi les radicaux alkyles en $C_1$-$C_8$, les halogènes et les radicaux alkoxy en $C_1$-$C_4$ étant entendu que, dans ce dernier cas, deux Y adjacents d'un même noyau aromatique peuvent former ensemble un groupement alkylidène dioxy dans lequel le groupe alkylidène contient de 1 à 2 atomes de carbone,
- X représente O ou NH,
- Z représente l'hydrogène ou un radical alkyle en $C_1$-$C_4$,
- n est un nombre entier compris entre 0 et 3 inclusivement,
- m est 0 ou 1,
- p représente un nombre entier compris entre 1 et 10, inclusivement.

Ces composés sont notamment décrits dans les demandes de brevet EP-A-0392883 ; EP-A-0660701 ; EP-A-0708108 ; EP-A-0711778 ; EP-A-711779.

De préférence, les dérivés siliciés utilisés dans le cadre de la présente invention appartiennent à la famille générale des silicones benzotriazoles qui est décrite notamment dans EP-A-0660701 .

Une famille de silicones benzotriazoles convenant particulièrement bien à la réalisation de la présente invention est celle regroupant les composés répondant aux formules (5) ou (6) suivantes :

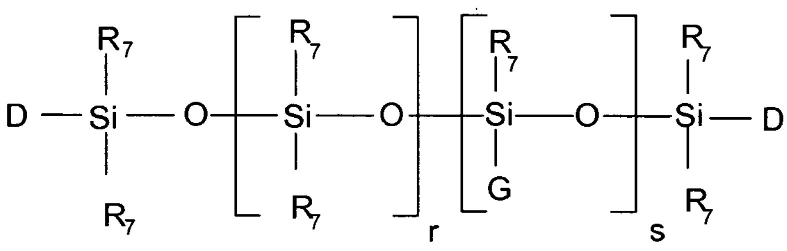

(5)

ou

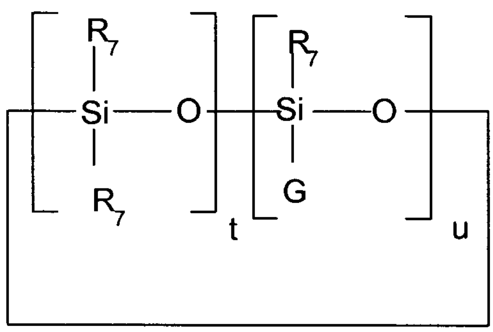

(6)

dans lesquelles :

- $R_7$, identiques ou différents, sont choisis parmi les radicaux alkyles en $C_1$-$C_{10}$, phényle, trifluoro-3,3,3 propyle et triméthylsilyloxy, au moins 80% en nombre des radicaux $R_7$ étant méthyle,
- D, identiques ou différents sont choisis parmi les radicaux $R_7$ et le radical G,
- r est un nombre entier compris entre 0 et 50 inclusivement, et s est un nombre entier compris entre 0 et 20 inclusivement, et si s = 0, au moins l'un des deux symboles D désigne G,
- u est un nombre entier compris entre 1 et 6 inclusivement, et t est un nombre entier compris entre 0 et 10 inclusivement, étant entendu que t + u est égal ou supérieur à 3,
- et le symbole G répond à la formule (2) ci-dessus.

Comme cela ressort de la formule (2) donnée ci-dessus, l'accrochage du chaînon $-(X)_m-(CH_2)_p-CH(Z)-CH_2-$ sur le motif benzotriazole, qui assure donc le raccordement dudit motif benzotriazole à l'atome de silicium de la chaîne siliconée, peut, selon la présente invention, se faire dans toutes les positions disponibles offertes par les deux noyaux aromatiques du benzotriazole :

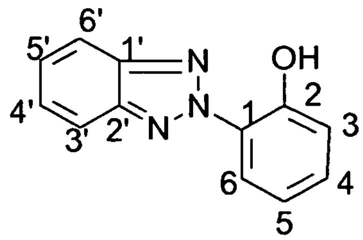

De préférence, cet accrochage se fait en position 3, 4, 5 (noyau aromatique portant la fonction hydroxy) ou 4' (noyau benzénique adjacent le cycle triazolé), et encore plus préférentiellement en position 3, 4 ou 5. Dans une forme préférée de réalisation de l'invention, l'accrochage se fait en position 3.

De même, l'accrochage du ou des motifs substituants Y peut se faire dans toutes les autres positions disponibles au sein du benzotriazole. Toutefois, de préférence, cet accrochage se fait en position 3, 4, 4', 5 et/ou 6. Dans une forme préférée de réalisation de l'invention, l'accrochage du motif Y se fait en position 5.

Dans les formules (5) et (6) ci-dessus, les radicaux alkyle peuvent être linéaires ou ramifiés et choisis notamment au sein des radicaux méthyle, éthyle, n-propyle, isopropyle, n-butyle, isobutyle, ter.-butyle, n-amyle, isoamyle, néopentyle, n-hexyle, n-heptyle, n-octyle, éthyl-2 hexyle et tert-octyle. Les radicaux alkyle $R_7$ préférés selon l'invention sont les radicaux méthyle, éthyle, propyle, n-butyle, n-octyle et éthyl-2 hexyle. Encore plus préférentiellement, les radicaux $R_7$ sont tous des radicaux méthyle.

Parmi les composés de formules (5) ou (6) ci-dessus, on préfère mettre en oeuvre ceux répondant à la formule (5), c'est-à-dire des diorganosiloxanes à chaîne courte linéaire.

Parmi les composés de formules (5) ci-dessus, on préfère mettre en oeuvre ceux pour lesquels les radicaux D sont tous les deux des radicaux $R_7$

Parmi les diorganosiloxanes linéaires rentrant dans le cadre de la présente invention, on préfère plus particulièrement les dérivés statistiques ou bien définis à blocs présentant au moins l'une, et encore plus préférentiellement l'ensemble, des caractéristiques suivantes :

- D est un radical $R_7$
- $R_7$ est alkyle et encore plus préférentiellement est méthyle,
- r est compris entre 0 et 15 inclusivement ; s est compris entre 1 et 10 inclusivement,
- n est non nul, et de préférence égal à 1, et Y est alors choisi parmi méthyle, tert.-butyle ou alcoxy en $C_1$-$C_4$,
- Z est hydrogène ou méthyle,
- m=0, ou [m=1 et X=O]
- p est égal à 1.

Une famille de silicones benzotriazoles convenant particulièrement bien à l'invention est celle définie par la formule générale (7) suivante :

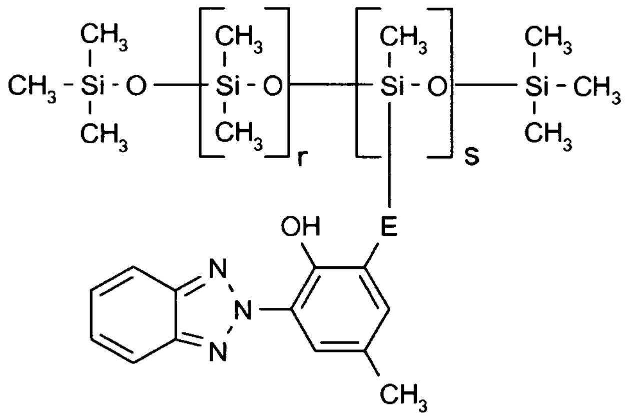

(7)

avec $0 \leq r \leq 10$,
$1 \leq S \leq 10$,
et où E représente le radical divalent :

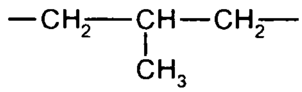

Dans une forme particulièrement préférée de réalisation de l'invention, la silicone benzotriazole est le composé Drométrizole Trisiloxane (nom CTFA) répondant à la formule suivante :

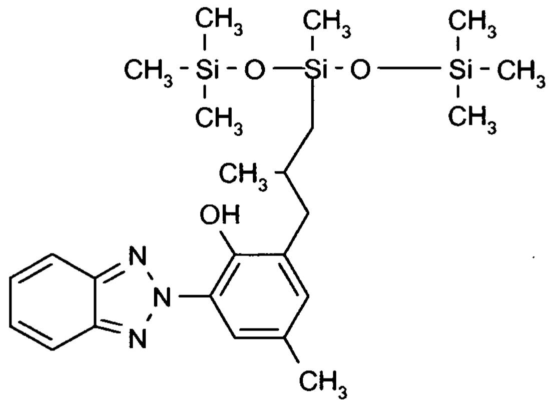

Des procédés convenant à la préparation des produits de formule (1), (5), (6) et (7) ci-dessus sont notamment décrits dans les brevets américains US3,220,972, US3,697,473, US4,340,709, US4,316,033, US4,328,346 et dans les demandes de brevet EP-A-0392883 et EP-A-0742 003.

Le dérivé silicié à fonction benzotriazole peut être présent dans les compositions selon l'invention à des teneurs allant de 0,1 à 10%, de préférence allant de 0,1 à 1 %, en poids, toujours par rapport au poids total de la composition.

## SUBSTANCES PARFUMANTES

Les parfums sont des compositions contenant notamment les matières premières decrites dans S. Arctander, Perfume and Flavor Chemicals (Montclair, N.J., 1969), dans S. Arctander, Perfume and Flavor Materials of Natural Origin (Elizabeth, N.J., 1960) et dans "Flavor and Fragrance Materials - 1991", Allured Publishing Co. Wheaton, III.

Il peut s'agir de produits naturels (huiles essentielles, absolus, résinoïdes, résines, concrètes) et/ou synthétiques (hydrocarbures terpéniques ou sesquiterpéniques, alcools, phénols, aldéhydes, cétones, éthers, acides, esters, nitriles, peroxydes, saturés ou insaturés, aliphatiques ou cycliques).

Selon la définition donnée dans la norme internationale ISO 9235 et adoptée par la Commission de la Pharmacopée Européenne, une huile essentielle est un produit odorant généralement de composition complexe, obtenu à partir d'une matière première végétale botaniquement définie, soit par entraînement à la vapeur d'eau, soit par distillation sèche, soit par un procédé mécanique approprié sans chauffage (Expression à froid). L'huile essentielle est le plus souvent séparée de la phase aqueuse par un procédé physique n'entraînant pas de changement significatif de la composition.

Modes d'obtention des huiles essentielles

Le choix de la technique dépend principalement de la matière première : son état originel et ses caractéristiques, sa nature proprement dit. Le rendement « huile essentielle/matière première végétale » peut être extrêmement variable selon les plantes : 15 ppm à plus de 20%. Ce choix conditionne les caractéristiques de l'huile essentielle, en particulier viscosité, couleur, solubilité, volatilité, enrichissement ou appauvrissement en certains constituants.

Entraînement à la vapeur d'eau

L'entrainement à la vapeur correspond à la vaporisation en présence de vapeur d'eau d'une substance peu miscible à l'eau. La matière première est mise en présence d'eau portée à ébullition ou de vapeur d'eau dans un alambic. La vapeur d'eau entraîne la vapeur d'huile essentielle qui est condensée dans le réfrigérant pour être récupérée en phase liquide dans un vase florentin (ou essencier) où l'huile essentielle est séparée de l'eau par décantation. On appelle « eau aromatique ou « hydrolat » ou « eau distillée florale », le distillat aqueux qui subsiste à l'entraînement à la vapeur d'eau, une fois la séparation de l'huile essentielle effectuée.

Distillation sèche

L'huile essentielle est obtenue par distillation des bois, écorces ou racines, sans addition d'eau ou de vapeur d'eau dans une enceinte fermée conçue pour que le liquide soit récupéré dans sa partie basse. L'huile de Cade constitue l'exemple le plus connu de ce mode d'obtention.

Expression à froid

Ce mode d'obtention ne s'applique qu'aux fruits agrumes (Citrus spp.) par des procédés mécaniques à température ambiante. Le principe de la méthode est le suivant : les zestes sont dilacérés et le contenu des poches sécrétrices qui ont été rompues est récupéré par un procédé physique. Le procédé classique consiste à exercer sous un courant d'eau une action abrasive sur toute la surface du fruit. Après élimination des déchets solides, l'huile essentielle est séparée de la phase aqueuse par centrifugation. La plupart des installations industrielles permettent en fait la récupération simultanée ou séquentielle des jus de fruits et de l'huile essentielle.

Caractères physico-chimiques.

Les huiles essentielles sont en général volatiles et liquides à température ambiante, ce qui les différencie des huiles dites fixes. Elles sont plus ou moins colorées et leur densité est en général inférieure à celle de l'eau. Elles ont un indice de réfraction élevée et la plupart dévient la lumière polarisée. Elles sont liposolubles et solubles dans les solvants organiques usuels, entraînables à la vapeur d'eau, très peu solubles dans l'eau.

Parmi les huiles essentielles utilisables selon l'invention, on peut citer celles obtenues à partir des plantes appartenant aux familles botaniques suivantes :

Abiétaceés ou Pinacées : conifères
Amaryllidacées
Anacardiacées
Anonacées : ylang ylang
Apiacées (par exemple les ombellifères) : aneth, angénique, coriandre, criste marine, carotte, persil
Aracées
Aristolochiacées
Astéracées : achilée, armoise, camomille, hélichryse
Bétulacées
Brassicacées
Burséracées : encens
Caryophyllacées

Canellacées
Césalpiniacées : copaïfera (copahu)
Chénopodacées
Cistacées : ciste
Cypéracées
Diptérocarpacées
Ericacées : gaulthérie (wintergreen)
Euphorbiacées
Fabacées
Geraniacées : géranium
Guttifères
Hamamélidacées
Hernandiacées
Hypéricacées : millepertuis
Iridacées
Juglandacées
Lamiacées : thym, origan, monarde, sarriette, basilic, marjolaines, menthes, patchouli, lavandes, sauges, cataire, romarin, hysope, mélisse, romarin
Lauracées : ravensara, laurier, bois de rose, cannelle, litséa
Liliacées : ail
Magnoliacées : magnolia
Malvacées
Méliacées
Monimiacées
Moracées : chanvre, houblon
Myricacées
Mysristicacées : muscade
Myrtacées : eucalyptus, tea tree, niaouli, cajeput, backousia, girofle, myrte Oléacées
Pipéracées : poivre
Pittosporacées
Poacées : citronnelle, lemongrass, vétiver
Polygonacées
Renonculacées
Rosacées : roses
Rubiacées
Rutacées : tous les citrus
Salicacées
Santalacées : santal
Saxifragacées
Schisandracées
Styracacées : benjoin
Thymélacées : bois d'agar
Tilliacées
Valérianacées : valériane, nard
Verbénacées : lantana, verveine
Violacées
Zingibéracées : galanga, curcuma, cardamome, gingembre
Zygophyllacées

On peut citer également les huiles essentielles extraites de fleurs (lis, lavande, rose, jasmin, yilang-ylang, néroli), de tiges et de feuilles (patchouli, géranium, petit-grain), de fruits (coriandre, anis, cumin, genièvre), d'écorces de fruits (bergamote, citron, orange), de racines (angélique, céleri, cardamome, iris, acore, gingembre), de bois (bois de pin, santal, gaïac, cèdre rose, camphre), d'herbes et de graminées (estragon, romarin, basilic, lemon grass, sauge, thym), d'aiguilles et de branches (épicéa, sapin, pin, pin nain), de résines et de baumes (galbanum, élémi, benjoin, myrrhe, oliban, opopanax).

Des exemples de substances parfumantes sont notamment : le géraniol, l'acétate de géranyle, le farnésol, le bornéol, l'acétate de bornyle, le linalol, l'acétate de linalyle, le propionate de linalyle, le butyrate de linalyle, le tétrahydrolinalol, le citronellol, l'acétate de citronellyle, le formate de citronellyle, le propionate de citronellyle, le dihydromyrcenol,

l'acétate de dihydromyrcenyle, le tétrahydromyrcenol, le terpinéol, l'acétate de terpinyle, le nopol, l'acétate de nopyle, le nérol, l'acétate de néryle, le 2-phényléthanol, l'acétate de 2-phényléthyle, l'alcool benzylique, l'acétate de benzyle, le salicylate de benzyle, l'acétate de styrallyle, le benzoate de benzyle, le salicylate d'amyle, le diméthylbenzyl-carbinol, l'acétate de trichlorométhylphénylcarbinyle, l'acétate de p-tert-butylcyclohexyle, l'acétate d'isononyle, l'acétate de vétivéryle, le vétivérol, l'alpha -hexylcinnamaldéhyde, le 2-méthyl-3-(p-tert-butylphényl)propanal, le 2-méthyl-3-(p-isopropylphényl)propanal, le 3-(p-tert-butylphényl)-propanal, le 2,4-diméthylcyclohex-3-enyl-carboxaldéhyde, l'acétate de tricyclodécènyle, le propionate de tricyclodécènyle, le 4-(4-hydroxy-4-méthylpentyl)-3-cyclohexènecarboxaldéhyde, le 4-(4-méthyl-3-pentènyl)-3-cyclohexènecarboxaldéhyde, le 4-acétoxy-3-pentyl-tétrahydropyrane, le 3-carboxyméthyl-2-pentylcyclopentane, la 2-n-4-heptylcyclopentanone, la 3-méthyl-2-pentyl-2-cyclopentènone, la menthone, la carvone, la tagétone, la géranyl acétone, le n-décanal, le n-dodécanal, le 9-décènol-1, l'isobutyrate de phénoxyéthyle, le phénylacétaldéhyde diméthyl-acétal, le phénylacétaldéhyde diéthylacétal, le géranonitrile, le citronellonitrile, l'acétate de cédryle, le 3-isocamphylcyclohexanol, le cédryl méthyl éther, l'isolongifolanone, l'aubépinonitrile, l'aubépine, l'héliotropine, la coumarine, l'eugénol, la vanilline, l'oxyde de diphényle, le citral, le citronellal, l'hydroxycitronellal, la damascone, les ionones, les méthylionones, les isométhylionones, la solanone, les irones, le cis-3-hexènol et ses esters, les muscs-indanes, les muscs-tétralines, les muscsisochromanes, les cétones macrocycliques, les muscs-macrolactones, le brassylate d'éthylène et leurs mélanges.

Selon un mode préféré de réalisation de l'invention, on utilise un mélange de différentes substances parfumantes qui engendrent en commun une note plaisante pour l'utilisateur.

On choisira de préférence les substances parfumantes de telles sorte qu'elle produisent des notes (tête, coeur et fond) dans les familles suivantes

les hespéridés,
les aromatiques,
les notes florales
les épicées,
les boisées
les gourmands
les chyprés,
les fougères
les cuirés.
les muscs

Les compositions parfumantes de l'invention contiennent de préférence de 2% à 40 % en poids de substance parfumante, mieux de 2% à 30% en poids, en particulier de 2% à 25% en poids par rapport au poids total

Le milieu cosmétiquement acceptable conforme à la présente invention contient de préférence au moins un alcool volatil et/ou une huile de silicone volatile et/ou une huile hydrocarbonée volatile et éventuellement de l'eau. De façon préférentielle le milieu de la composition contient de l'eau dans une quantité allant de préférence de 0,01 % à 50 % et plus préférentiellement, de 0,5 % à 25 % en poids par rapport au poids total de la composition.

Par " volatil", on entend au sens de l'invention toute molécule susceptible de s'évaporer au contact de la peau ou de la fibre kératinique en moins d'une heure, à température ambiante et pression atmosphérique. Le ou les composés volatils de l'invention sont liquides à température ambiante, ont une pression de vapeur non nulle, à température ambiante et pression atmosphérique, allant en particulier de 0,13 Pa à 40 000 Pa ($10^{-3}$ à 300 mm de Hg), en particulier allant de 1,3 Pa à 13 000 Pa (0,01 à 100 mm de Hg), et plus particulièrement allant de 1,3 Pa à 1300 Pa (0,01 à 10 mm de Hg).

## ALCOOLS VOLATILS

Par « alcool volatil », on entend tout composé ayant au moins un groupe hydroxyle et où plus de 95% en poids du composé est susceptible de s'évaporer en moins d'une heure à température ambiante (25°C) et pression atmosphérique (760 mmHg) au contact d'une matière kératinique comme la peau ou les cheveux.

Les alcools volatils conformes à la présente invention sont choisis de préférence parmi monoalcools inférieurs en $C_1$-$C_5$ peuvent être choisis parmi le méthanol, l'éthanol, propanol, l'isopropanol, le n-butanol, l'isobutanol, le t-butanol et plus particulièrement l'éthanol. Leur viscosité à 20°C, mesurée avec un appareil HAAKE Rheostress 600 avec un rotor de 60 mm de diamètre, un angle de 2° à une vitesse de cisaillement de 200 $s^{-1}$ est de préférence de 0,3 à 3 mPa.s.

Le ou les alcools volatils sont présents de préférence dans des quantités allant de 40 à 80% et plus préférentiellement dans des quantités allant de 55 à 80% en poids par rapport au poids total de la composition.

## HUILES SILICONEES VOLATILES

Comme huiles siliconées volatiles, on peut citer par exemple les huiles de silicones linéaires ou cycliques volatiles, notamment celles ayant une viscosité ≤ 6 centistokes ($6.10^{-6}$ $m^2/s$), et ayant notamment de 2 à 10 atomes de

silicium, ces silicones comportant éventuellement des groupes alkyles ou alkoxy ayant de 1 à 22 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

L'huile ou les huiles siliconées volatiles sont présentes de préférence de 10 à 80% par rapport au poids total de la composition.

## HUILES HYDROCARBONEES VOLATILES

Les huiles hydrocarbonées volatiles peuvent être choisies parmi les huiles hydrocarbonées ayant de 8 à 16 atomes de carbones, et notamment les alcanes ramifiés en C8-C16 comme les isoalcanes en $C_8$-$C_{16}$ d'origine pétrolière (appelées aussi isoparaffines) comme l'isododécane (encore appelé 2,2,4,4,6-pentaméthylheptane), l'isodécane, l'isohexadécane, les huiles vendues sous les noms commerciaux d'Isopars ou de Permetyls, les esters ramifiés en $C_8$-$C_{16}$ le néopentanoate d'iso-hexyle, et leurs mélanges. D'autres huiles hydrocarbonées volatiles comme les distillats de pétrole, notamment ceux vendus sous la dénomination Shell Solt par la société SHELL, peuvent aussi être utilisées. Selon un mode de réalisation, le solvant volatil est choisi parmi les huiles volatiles hydrocarbonées ayant de 8 à 16 atomes de carbone et leurs mélanges.

Selon un mode particulièrement préféré de l'invention, on utilisera comme huile hydrocarbonée un alcane linéaire volatil.

## ALCANES LINEAIRES VOLATILS

La composition selon l'invention contient un ou plusieurs alcane(s) linéaire(s) volatil(s). Par « un ou plusieurs alcane(s) linéaire(s) volatil(s) », on entend indifféremment « une ou plusieurs huile alcane(s) linéaire(s) volatile(s) ».

Un alcane linéaire volatil convenant à l'invention est liquide à température ambiante (environ 25°C) et à la pression atmosphérique (760 mm Hg).

Par « alcane linéaire volatil » convenant à l'invention, on entend un alcane linéaire cosmétique, susceptible de s'évaporer au contact de la peau en moins d'une heure, à température ambiante (25°C) et pression atmosphérique (760 mm Hg, c'est-à-dire 101 325 Pa), liquide à température ambiante, ayant notamment une vitesse d'évaporation allant de 0,01 à 15 mg/cm$^2$/min, à température ambiante (25°C) et pression atmosphérique (760 mm Hg).

De façon préférée, les « alcanes linéaires volatils » convenant à l'invention présentent une vitesse d'évaporation allant de 0,01 à 3,5 mg/cm$^2$/min, à température ambiante (25°C) et pression atmosphérique (760 mm Hg).

De façon préférée, les « alcanes linéaires volatils » convenant à l'invention présentent une vitesse d'évaporation allant de 0,01 à 1,5 mg/cm$^2$/min, à température ambiante (25°C) et pression atmosphérique (760 mm Hg).

De façon plus préférée, les « alcanes linéaires volatils » convenant à l'invention présentent une vitesse d'évaporation allant de 0,01 à 0,8 mg/cm$^2$/min, à température ambiante (25°C) et pression atmosphérique (760 mm Hg).

De façon encore préférée, les « alcanes linéaires volatils » convenant à l'invention présentent une vitesse d'évaporation allant de 0,01 à 0,3 mg/cm$^2$/min, à température ambiante (25°C) et pression atmosphérique (760 mm Hg).

De façon encore préférée, les « alcanes linéaires volatils » convenant à l'invention présentent une vitesse d'évaporation allant de 0,01 à 0,12 mg/cm$^2$/min, à température ambiante (25°C) et pression atmosphérique (760 mm Hg).

La vitesse d'évaporation d'un alcane volatil conforme à l'invention (et plus généralement d'un solvant volatil) peut être notamment évaluée au moyen du protocole décrit dans WO 06/013413, et plus particulièrement au moyen du protocole décrit ci-après.

On introduit dans un cristallisoir (diamètre : 7 cm) placé sur une balance se trouvant dans une enceinte d'environ 0,3 m$^3$ régulée en température (25°C) et en hygrométrie (humidité relative 50 %) 15 g de solvant hydrocarboné volatil.

On laisse le liquide s'évaporer librement, sans l'agiter, en assurant une ventilation par un ventilateur (PAPST-MOTOREN, référence 8550 N, tournant à 2700 tours/minute) disposé en position verticale au-dessus du cristallisoir contenant le solvant hydrocarboné volatil, les pales étant dirigées vers le cristallisoir, à une distance de 20 cm par rapport au fond du cristallisoir.

On mesure à intervalles de temps réguliers la masse de solvant hydrocarboné volatil restante dans le cristallisoir.

On obtient alors le profil d'évaporation du solvant en traçant la courbe de la quantité de produit évaporé (en mg/cm$^2$) en fonction du temps (en min). Puis on calcule la vitesse d'évaporation qui correspond à la tangente à l'origine de la courbe obtenue. Les vitesses d'évaporation sont exprimées en mg de solvant volatil évaporé par unité de surface (cm$^2$) et par unité de temps (minute).

Selon un mode de réalisation préféré, les « alcanes linéaires volatils » convenant à l'invention ont une pression de vapeur (appelée également pression de vapeur saturante) non nulle, à température ambiante, en particulier une pression de vapeur allant de 0,3 Pa à 6000 Pa.

De façon préférée, les « alcanes linéaires volatils » convenant à l'invention ont une pression de vapeur allant de 0,3 à 2000 Pa, à température ambiante (25°C).

De façon préférée, les « alcanes linéaires volatils » convenant à l'invention ont une pression de vapeur allant de 0,3 à 1000 Pa, à température ambiante (25°C)

De façon plus préférée, les « alcanes linéaires volatils » convenant à l'invention ont une pression de vapeur allant de 0,4 à 600 Pa, à température ambiante (25°C).

De façon préférée, les « alcanes linéaires volatils » convenant à l'invention ont une pression de vapeur allant de 1 à 200 Pa, à température ambiante (25°C).

De façon encore préférée, les « alcanes linéaires volatils » convenant à l'invention ont une pression de vapeur allant de 3 à 60 Pa, à température ambiante (25°C).

Selon un mode de réalisation, un alcane linéaire volatil convenant à l'invention peut présenter un point éclair compris dans l'intervalle variant de 30 à 120°C, et plus particulièrement de 40 à 100°C. Le point éclair est en particulier mesuré selon la Norme iso 3679.

Selon un mode de réalisation, un alcane convenant à l'invention peut être un alcane linéaire volatil comprenant de 7 à 14 atomes de carbone.

De façon préférée, les « alcanes linéaires volatils » convenant à l'invention comprennent de 8 à 14 atomes de carbone.

De façon préférée, les « alcanes linéaires volatils » convenant à l'invention comprennent de 9 à 14 atomes de carbone.

De façon préférée, les « alcanes linéaires volatils » convenant à l'invention comprennent de 10 à 14 atomes de carbone.

De façon préférée, les « alcanes linéaires volatils » convenant à l'invention comprennent de 11 à 14 atomes de carbone.

Selon un mode de réalisation avantageux, les « alcanes linéaires volatils » convenant à l'invention présentent une vitesse d'évaporation, telle que définie plus haut, allant de 0,01 à 3,5 mg/cm$^2$/min, à température ambiante (25°C) et pression atmosphérique (760 mm Hg), et comprennent de 8 à 14 atomes de carbone.

Un alcane linéaire volatil convenant à l'invention peut être avantageusement d'origine végétale.

De préférence, l'alcane linéaire volatil ou le mélange d'alcanes linéaires volatils présent dans la composition selon l'invention comprend au moins un isotope $^{14}C$ du carbone (carbone 14), en particulier l'isotope $^{14}C$ peut être présent en un ratio $^{14}C$ / $^{12}C$ supérieur ou égal à $1.10^{-16}$, de préférence supérieur ou égal à $1.10^{-15}$, de préférence encore supérieur ou égal $7,5.10^{-14}$, et mieux supérieur ou égal $1,5.10^{-13}$. De préférence, le ratio $^{14}C$ / $^{12}C$ va de $6.10^{-13}$ à $1,2.10^{-12}$.

La quantité de d'isotopes $^{14}C$ dans l'alcane linéaire volatil ou le mélange d'alcanes linéaires volatils peut être déterminée par des méthodes connues de l'homme du métier telles que la méthode de comptage de Libby, la spectrométrie à scintillation liquide ou encore la spectrométrie de masse à accélération (Accelerator Mass Spectrometry).

Un tel alcane peut être obtenu, directement ou en plusieurs étapes, à partir d'une matière première végétale comme une huile, un beurre, une cire, etc.

A titre d'exemple d'alcanes convenant à l'invention, on peut mentionner les alcanes décrits dans les demandes de brevets de la société Cognis WO 2007/068371, ou WO2008/155059 (mélanges d'alcanes distincts et différant d'au moins un carbone). Ces alcanes sont obtenus à partir d'alcools gras, eux-mêmes obtenus à partir d'huile de coprah ou de palme.

A titre d'exemple d'alcanes linéaires convenant à l'invention, on peut citer le n- heptane ($C_7$), le n-octane ($C_8$), le n-nonane ($C_9$), le n-décane ($C_{10}$), le n-undécane ($C_{11}$), le n-dodécane ($C_{12}$), le n-tridécane ($C_{13}$), le n-tétradecane ($C_{14}$), et leurs mélanges. Selon un mode de réalisation particulier, l'alcane linéaire volatil est choisi parmi le n-nonane, le n-undécane, le n-dodécane, le n-tridécane, le n-tétradécane, et leurs mélanges.

Selon un mode préféré, on peut citer les mélanges de n-undécane ($C_{11}$) et de n-tridécane ($C_{13}$) obtenus aux exemples 1 et 2 de la demande WO2008/155059 de la Société Cognis.

On peut également citer le n-dodécane ($C_{12}$) et le n-tétradécane ($C_{14}$) vendus par Sasol respectivement sous les références PARAFOL 12-97 et PARAFOL 14-97, ainsi que leurs mélanges.

On pourra utiliser l'alcane linéaire volatil seul.

On pourra alternativement ou préférentiellement utiliser un mélange d'au moins deux alcanes linéaires volatils distincts, différant entre eux d'un nombre de carbone n d'au moins 1, en particulier différant entre eux d'un nombre de carbone de 1 ou de 2.

Selon un premier mode de réalisation, on utilise un mélange d'au moins deux alcanes linéaires volatils distincts comportant de 10 à 14 atomes de carbone et différant entre eux d'un nombre de carbone d'au moins 1. A titre d'exemples, on peut citer notamment les mélanges d'alcanes linéaires volatils $C_{10}/C_{11}$, $C_{11}/C_{12}$, ou $C_{12}/C_{13}$.

Selon un autre mode de réalisation, on utilise un mélange d'au moins deux alcanes linéaires volatils distincts comportant de 10 à 14 atomes de carbone et différant entre eux d'un nombre de carbone d'au moins 2. A titre d'exemples,

on peut citer notamment les mélanges d'alcanes linéaires volatils $C_{10}/C_{12}$, ou $C_{12}/C_{14}$, pour un nombre de carbone n pair et le mélange $C_{11}/C_{13}$ pour un nombre de carbone n impair.

Selon un mode préféré, on utilise un mélange d'au moins deux alcanes linéaires volatils comportant de 10 à 14 atomes de carbone distincts et différant entre eux d'un nombre de carbone d'au moins 2, et en particulier un mélange d'alcanes linéaires volatils $C_{11}/C_{13}$ ou un mélange d'alcanes linéaires volatils $C_{12}/C_{14}$.

D'autres mélanges associant plus de 2 alcanes linéaires volatils selon l'invention, tels que par exemple un mélange d'au moins 3 alcanes linéaires volatils comportant de 7 à 14 atomes de carbone distincts et différant entre eux d'un nombre de carbone d'au moins 1, font également partie de l'invention, mais les mélanges de 2 alcanes linéaires volatils selon l'invention sont préférés (mélanges binaires), lesdits 2 alcanes linéaires volatils représentant de préférence plus de 95% et mieux plus de 99% en poids de la teneur totale en alcanes linéaires volatils dans le mélange. Selon un mode particulier de l'invention, dans un mélange d'alcanes linéaires volatils, l'alcane linéaire volatil ayant le nombre de carbone le plus petit est majoritaire dans le mélange.

Selon un autre mode de l'invention, on utilise un mélange d'alcanes linéaires volatils dans lequel l'alcane linéaire volatil ayant le nombre de carbone le plus grand est majoritaire dans le mélange.

A titre d'exemples de mélanges convenant à l'invention, on peut citer notamment les mélanges suivants :

- de 50 à 90% en poids, de préférence de 55 à 80% en poids, préférentiellement encore de 60 à 75% en poids d'alcane linéaire volatil en $C_n$ avec n allant de 7 à 14
- de 10 à 50% en poids, de préférence de 20 à 45% en poids, de préférence de 24 à 40% en poids, d'alcane linéaire volatil en $C_{n+x}$ avec x supérieur ou égal à 1, de préférence x=1 ou x=2, avec n+x compris entre 10 et 14, par rapport au poids total des alcanes dans ledit mélange.

En particulier, ledit mélange d'alcanes selon l'invention contient :

- moins de 2% en poids, de préférence moins de 1% en poids d'hydrocarbures ramifiés,
- et/ou moins de 2% en poids, de préférence moins de 1% en poids d'hydrocarbures aromatiques,
- et/ou moins de 2% en poids, de préférence moins de 1% en poids et préférentiellement moins de 0,1% en poids d'hydrocarbures insaturés dans le mélange.

Plus particulièrement, un alcane linéaire volatil convenant à l'invention peut être mis en oeuvre sous la forme d'un mélange n-undécane/n-tridécane.

En particulier, on utilisera un mélange d'alcanes linéaires volatils comprenant :

- de 55 à 80% en poids, de préférence de 60 à 75% en poids d'alcane linéaire volatil en $C_{11}$ (n-undécane)
- de 20 à 45% en poids, de préférence de 24 à 40% en poids d'alcane linéaire volatil en $C_{13}$ (n-tridécane)

par rapport au poids total des alcanes dans ledit mélange.

Selon un mode de réalisation particulier, le mélange d'alcanes est un mélange n-undécane/n-tridécane. En particulier un tel mélange peut être obtenu selon l'exemple 1 ou l'exemple 2 du WO2008/155059.

Selon un autre mode de réalisation particulier, on utilise le n-dodécane vendu sous la référence PARAFOL 12-97 par SASOL.

Selon un autre mode de réalisation particulier, on utilise le n-tétradécane vendu sous la référence PARAFOL 14-97 par SASOL.

Selon encore un autre mode de réalisation, on utilise un mélange de n-dodécane et de n-tétradécane.

## ADDITIFS

La composition de l'invention peut comprendre, en outre, tout additif usuellement utilisé dans le domaine des parfums choisi notamment parmi les antioxydants, des corps gras comme des huiles (huiles végétales, minérales ou synthétiques comme les esters, les perfluoroéthers), les actifs cosmétiques ou dermatologiques comme par exemple les émollients ou adoucissants comme les huiles d'amande douce, de noyau d'abricot, les agents hydratants comme la glycérine, les agents apaisants comme l'$\alpha$-bisabolol, l'allantoïne, aloes vera ; les vitamines et leurs dérivés, les acides gras essentiels, les agents répulsifs contre les insectes, les propulseurs, les peptisants, des charges, des co-solvants, des filtres UV autres que ceux que les filtres UVA hydrophiles, des stabilisants ou conservateurs, des colorants, des nacres, des paillettes et leurs mélanges. Quand ils sont présents dans la composition de l'invention, ces additifs peuvent être présents en une quantité allant de 0,001 à 10 % et mieux de 0,01 à 5% en poids par rapport au poids total de la composition.

Parmi les co-solvants utilisables selon l'invention, on peut citer l'octyldodécanol, le tri-éthylcitrate, le dicaprylylcarbonate, l'isononanoate d'isononyle, le myristate et palmitate d'isopropyle, le palmitate de 2-éthylhexyle.

Parmi les filtres UV additionnels, on peut citer les filtres organiques filtrant les radiations UVA et/ou UVB comme

- les dérivés de dibenzoylméthane comme le Butyl Methoxydibenzoylmethane vendu notamment sous le nom commercial « PARSOL 1789 » par par DSM NUTRITIONAL PRODUCTS,
- les dérivés de benzophénone hydrophobes comme le Benzophenone-3 ou Oxybenzone vendu sous le nom commercial « UVINUL M40 » par BASF,
- les dérivés d'aminobenzophénone comme le 2-(4-diethylamino-2-hydroxybenzoyl)-benzoate de n-hexyle vendu sous le nom commercial« UVINUL A +» par BASF,
- les dérivés d'acide salicylique comme l'Homosalate vendu sous le nom « Eusolex HMS » par Rona/EM Industries et l'Ethylhexyl Salicylate vendu sous le nom « NEO HELIOPAN OS » par SYMRISE.
- les dérivés d'acide cinnamique comme l'Ethylhexyl Methoxycinnamate vendu notamment sous le nom commercial « PARSOL MCX » par DSM NUTRITIONAL PRODUCTS,

La composition de l'invention peut comprendre, en outre des colorants solubles dans le support de ladite composition

Comme colorants solubles conformes à l'invention, on peut citer les colorants hydrosolubles ou hydrophiles tels que:

le caramel, Yellow 5 , Acid Blue 9/ Blue 1, Green 5, Green 3 / Fast Green FCF 3, Orange 4, Red 4 / Food Red 1, Yellow 6, Acid Red 33 / Food Red 12, Red 40, le carmine de cochenille (CI 15850, CI 75470), Ext. Violet 2, Red 6-7, Ferric Ferrocyanide, Ultramarines, Acide Yellow 3 / Yellow 10, Acid Blue 3, Yellow 10.

Le ou les colorant (s) soluble (s) conformes à l'invention sont de préférence présents dans des quantités allant de $10^{-5}$ à 1 % du poids total de la composition, de préférence de $10^{-4}$ à 0,1% du poids total de la composition.

Comme stabilisants de la couleur de parfums, on citera le Tris(tétraméthylhydroxypipéridinol) citrate tel que produit vendu sous le nom « TINOGUARD Q » par la société CIBA-GEIGY, le Sodium Benzotriazolyl Butylphenol Sulfonate comme le produit vendu sous le nom « TINOGUARD HS » par la société CIBA-GEIGY ; le Benzotriazolyl dodécyl p-Cresol comme le produit vendu sous le nom « TINOGUARD TL » par la société CIBA-GEIGY. comme le produit vendu sous le nom commercial « CIBAFAST H LIQUID » par la société CIBA-GEIGY. Bumetrizole comme le produit vendu sous le nom « TINOGUARD AS » par la société CIBA-GEIGY.

Selon une forme particulière de l'invention, on utilisera en plus au moins un antioxydant et/ou au moins un agent peptisant de manière à améliorer la limpidité de la composition et/ou diminuer voire supprimer les phénomènes de précipitation à froid pouvant êtres causés par certains parfums et/ou améliorer la stabilité de la composition au stockage.

Parmi les antioxydants, on peut citer par exemple le BHA (tert-butyl-4-hydroxyanisole), le BHT (2,6-di-tert-butyl-p-crésol), les tocophérols comme la vitamine E et ses dérivés tels que l'acétate de tocophéryle. Ils sont utilisés à des concentrations allant de 0.01 % à 1 % par rapport au poids total de la composition.

Parmi les peptisants utilisables selon l'invention, on utilisera plus particulièrement l'huile de ricin hydrogénée oxyéthylénée.à 60 moles d'oxyde d'ethylène : Nom INCI : PEG-60 HYDROGENATED CASTOR OIL comme les produit vendus sous les noms commerciaux CREMOPHOR RH60 ou CREMOPHOR C040. par la société BASF. Ils sont utilisés à des concentrations allant de 0.1% à 2 fois la concentration en concentré de parfum par rapport au poids total de la composition

Bien entendu l'homme du métier veillera à choisir les éventuels additifs complémentaires et/ou leur quantité de telle manière que les propriétés avantageuses de la composition selon l'invention ne soient pas ou substantiellement pas altérées par l'adjonction envisagée.

## FORMES GALENIQUES

L'invention s'applique non seulement aux produits parfumants mais aussi aux produits de soin, de traitement de la peau, y compris du cuir chevelu, et des lèvres, contenant une substance odorante. La composition selon l'invention peut ainsi constituer une composition de parfumage, de soin, de traitement des matières kératiniques, et notamment se présenter sous forme d'eau fraîche, d'eau de toilette, d'eau de parfum, de lotion après-rasage, d'eau de soin, d'huile de soin siliconée ou hydrosiliconée. Elle peut également se présenter sous la forme d'une lotion bi-phasique parfumée (phase eau de toilette/phase huile hydrocarbonée et/ou huile siliconée).

La composition selon l'invention peut être fabriquée par les procédés connus, généralement utilisés dans le domaine des formulations parfumées.

Les compositions selon l'invention peuvent être conditionnées sous forme de flacons.

La composition parfumante de l'invention peut être diffusée selon différents systèmes comme des sprays, des aérosols, des dispositifs piézoélectriques.

Elles peuvent également être appliquées sous forme de fines particules au moyen de dispositifs de pressurisation mécanique ou à gaz propulseur. Les dispositifs conformes à l'invention sont bien connus de l'homme de l'art et comprennent les flacons-pompes ou "sprays", les récipients aérosols comprenant un propulseur ainsi que les pompes aérosols utilisant l'air comprimé comme propulseur. Ces derniers sont décrits dans les brevets US 4,077,441 et US 4,850,517.

Les compositions conditionnées en aérosol conformes à l'invention contiennent en général des agents propulseurs conventionnels tels que par exemple le diméthyléther, l'isobutane, le n-butane, le propane.

Les compositions parfumantes sont de préférence transparentes Leur transparence se mesure par une turbidité allant de 1 à 200 NTU et de préférence de 10 à 90 NTU turbidité mesurée à 24 heures au turbidimètre portatif HACH - Modèle 2100 P.

Les compositions selon l'invention sont selon une forme particulière de l'invention des lotions et ont de préférence une viscosité allant de 10 à 120 UD et plus préférentiellement de 30 à 120 UD, encore plus préférentiellement de 40 à 80 UD ; la viscosité étant mesurée au Rhéomat TVe-05 , à 25°c, vitesse de rotation 200ts/min, mobile 1, 10 min. Ces faibles viscosités permettent de conditionner les compositions de l'invention au moyen de dispositifs de pressurisation mécanique ou à gaz propulseur de manière à être appliquées sous forme de fines particules (vaporisation).

L'invention va maintenant être décrite en référence aux exemples suivants donnés à titre illustratif et non limitatif. Dans ces exemples, sauf indication contraire, les quantités sont exprimées en pourcentages pondéraux. On a réalisé les formulations parfumées suivantes ; les quantités sont indiquées en pourcentages en poids :

**Exemples**

Les essais ont été réalisés selon le protocole de mesure décrit sur EP1897592 A1, mais sur différentes couleurs, afin de prouver le pouvoir de protection de

la silicone benzotriazole de formule (1) : Drometrizole Trisiloxane (Mexoryl XL) sur deux types de couleurs très différentes :

YELLOW 5 / CI 19140
EXT. VIOLET 2 / CI 60730

**Principe**

Le principe de ces essais consiste à exposer les produits à tester à une source lumineuse irradiante dont la répartition spectrale est parfaitement définie et l'énergie émise parfaitement quantifiée. Les sources lumineuses couramment utilisées sont des lampes au xénon émettant au travers :

- un filtre en quartz platiné qui détourne le rayonnement infra rouge et l'élimine vers le haut de l'appareil
- et un filtre en verre, qui en absorbant le rayonnement ultra violet court permet de simuler le rayonnement reçu derrière une vitrine d'exposition.

**Matériel**

On utilise un appareil CPS Sun -test :

- L'éclairement fourni par une lampe au xénon entre 300 et 800nm est fixé à 765W/$m^2$ (valeur réglée par le constructeur)
- La filtration optique est assurée par d'un filtre quartz avec revêtement IR et un verre à vitres spécial)

On observe ensuite la couleur de chaque flacon avant et après exposition à la lumière, à l'oeil nu et à l'aide d'un spectrophotomètre MINOLTA CM3600-d. On quantifie la couleur obtenue. Les résultats sous forme de moyennes sont exprimés dans le système (L*, a*, b*) dans lequel L* représente la luminance, a* représente l'axe rouge-vert (-a* = vert, +a* = rouge) et b* représente l'axe jaune-bleu (-b* =bleu, +b* =jaune). Ainsi, a* et b* expriment la couleur de la composition. La variation de couleur de l'eau de toilette après 16h de suntest est évaluée à l'oeil, mais également par le spectrocolorimètre par le $\Delta$E, qui correspond à la racine des sommes au carré des valeurs L* a* b* du spectrocolorimètre. Plus la valeur du $\Delta$E est élevée puis la dégradation de la couleur est importante.

On réalise et on teste les formulations suivantes :

| Ingrédients | Ex 1 (hors invention) | Ex 2 (hors invention | Ex 3 (hors invention) | Ex 4 (invention) |
|---|---|---|---|---|
| Parfum OTB Flanker | 10,00 | 10,00 | 10,00 | 10,00 |
| Colorant | X | X | X | X |
| Octocrylene (Uvinul N539) | - | 0,20 | 0,20 | - |
| Butylmethoxydibenzoylmethane (Parsol 1789) | - | 0,20 | - | - |
| Drometrizole Trisiloxane (Mexoryl XL) | - | - | 0,20 | 0,40 |
| Ethanol | 75.00 | 75.00 | 75.00 | 75.00 |
| Eau | qsp 100 | qsp 100 | qsp 100 | qsp 100 |

Les colorants sont utilisés dans les quantités X suivantes :

| | |
|---|---|
| YELLOW 5 / CI 19140 | 5,25 ppm |
| EXT. VIOLET 2 / CI 60730 | 1,75 ppm |

Tableau 1

| Colorant | Ex 1 (hors invention) | Ex 2 (hors invention) | Ex 3 (hors invention) | EX 4 (invention) |
|---|---|---|---|---|
| Colorant Jaune | ΔE=16.6 (décoloré) | ΔE=10,2 (Jaune) | ΔE=10,4 (Jaune) | ΔE=4,48 (Jaune) |
| Colorant Violet | ΔE = 5,2 (décoloré) | ΔE = 2,3 (décoloré) | ΔE = 1,8 (décoloré) | ΔE = 0,5 (Violet) |

On observe que la composition **4** selon l'invention comprenant le filtre silicone benzotriazole de formule (I) (ie Drometrizole Trisiloxane) sans composé β,β'-diphénylacrylate d'alkyle ni composé α-cyano-β,β'-diphénylacrylate d'alkyle (ie octocrylene) présente une couleur plus stable dans le temps que

- la composition **1** sans filtre
- la composition **2** associant l'octocrylène à un autre filtre UVA
- la composition **3** associant l'octocrylène avec la silicone benzotriazole de formule (1) : Drometrizone Trisiloxane

On en déduit que la présence de l'octocrylène a tendance à inhiber l'effet photoprotecteur sur la couleur du dérivé de benzylidène camphre sulfonique ou de la silicone benzotriazole de formule (1) sur la couleur du parfum.

## Revendications

**1.** Composition parfumante colorée comprenant dans un milieu cosmétiquement acceptable :

a) au moins 2% en poids d'une substance parfumante par rapport au poids total de la composition ;
b) au moins une silicone benzotriazole de formule (1) suivante :

$$O_{(3-a)/2}Si(R)_a\text{-}G \qquad (1)$$

dans laquelle :

- R représente un radical alkyle en $C_1$-$C_{10}$ éventuellement halogéné ou un radical phényle ou un radical triméthylsilyloxy,
- a est un nombre entier choisi entre 0 et 3 inclusivement,
- et le symbole G désigne un radical monovalent lié directement à un atome de silicium, et qui répond à la formule (2) suivante :

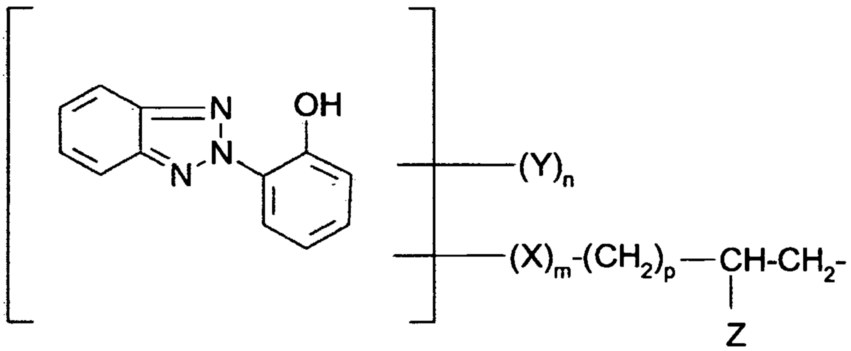

(2)

dans laquelle :

- Y, identiques ou différents, sont choisis parmi les radicaux alkyles en $C_1$-$C_8$, les halogènes et les radicaux alkoxy en $C_1$-$C_4$ étant entendu que, dans ce dernier cas, deux Y adjacents d'un même noyau aromatique peuvent former ensemble un groupement alkylidène dioxy dans lequel le groupe alkylidène contient de 1 à 2 atomes de carbone,
- X représente O ou NH,
- Z représente l'hydrogène ou un radical alkyle en $C_1$-$C_4$,
- n est un nombre entier compris entre 0 et 3 inclusivement,
- m est 0 ou 1,
- p représente un nombre entier compris entre 1 et 10, inclusivement ;

c) au moins un colorant soluble dans ledit milieu ; ladite composition ne contenant pas de composé β,β'-diphénylacrylate d'alkyle ou α-cyano-β,β'-diphénylacrylate d'alkyle.

2. Composition selon la revendication 1, où le dérivé silicié de benzotriazole de formule (I) est choisi parmi les composés répondant aux formules (5) ou (6) suivantes :

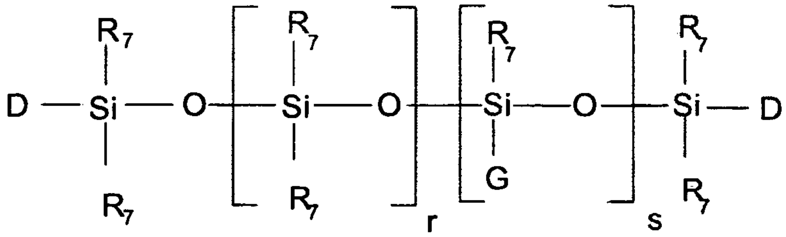

(5)

ou

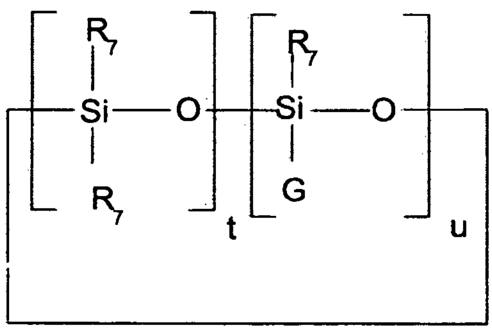

(6)

dans lesquelles :

- $R_7$, identiques ou différents, sont choisis parmi les radicaux alkyles en $C_1$-$C_{10}$, phényle, trifluoro-3,3,3 propyle et triméthylsilyloxy, au moins 80% en nombre des radicaux $R_7$ étant méthyle,
- D, identiques ou différents sont choisis parmi les radicaux $R_7$ et le radical G,
- r est un nombre entier compris entre 0 et 50 inclusivement, et s est un nombre entier compris entre 0 et 20 inclusivement, et si s = 0, au moins l'un des deux symboles D désigne G,
- u est un nombre entier compris entre 1 et 6 inclusivement, et t est un nombre entier compris entre 0 et 10 inclusivement, étant entendu que t + u est égal ou supérieur à 3,
- et le symbole G répond à la formule (2) ci-dessus.

3. Composition selon la revendication 2, où le dérivé silicié de benzotriazole de formule (I) est choisi parmi les composés répondant à la formule (5).

4. Composition selon la revendication **2 ou 3,** où le dérivé silicié de benzotriazole de formule (I) est choisi parmi les composés répondant à la formule (5) pour lesquels les radicaux D sont tous les deux des radicaux $R_7$.

5. Composition selon l'une quelconque des revendications **2 à 4,** où le dérivé silicié de benzotriazole de formule (5) présentant au moins l'une, et encore plus préférentiellement l'ensemble, des caractéristiques suivantes :

   - D est un radical $R_7$
   - $R_7$ est alkyle et encore plus préférentiellement est méthyle,
   - r est compris entre 0 et 15 inclusivement ; s est compris entre 1 et 10 inclusivement,
   - n est non nul, et de préférence égal à 1, et Y est alors choisi parmi méthyle, tert.-butyle ou alcoxy en $C_1$-$C_4$,
   - Z est hydrogène ou méthyle,
   - m=0, ou [m=1 et X=O]
   - p est égal à 1.

6. Composition selon l'une quelconque des revendications 1 à 6, où le composé de formule (1) est choisi parmi les composés répondant à la formule (7) suivante :

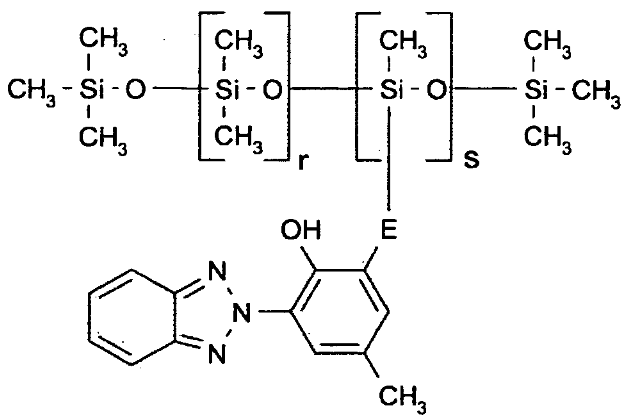

(7)

avec $0 \leq r \leq 10$,

$$1 \leq s \leq 10,$$

et où E représente le radical divalent :

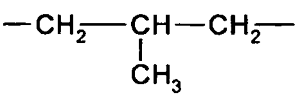

7. Composition selon la revendication 6, où le composé de formule (7) est le composé Drométrizole Trisiloxane répondant à la formule suivante :

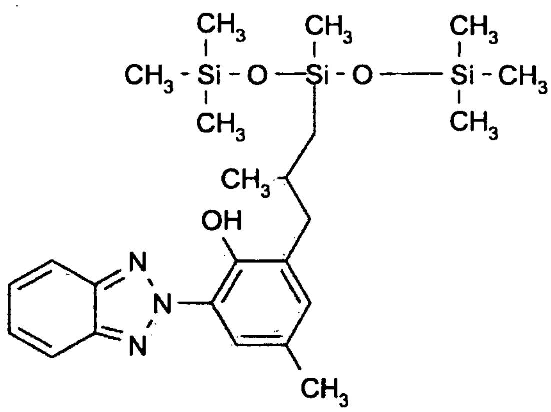

8. Composition selon l'une quelconque des revendications 1 à **7,** comprenant en outre, au moins un additif choisi parmi les antioxydants, des corps gras comme des huiles (huiles végétales, minérales ou synthétiques comme les esters, les perfluoroéthers), les actifs cosmétiques ou dermatologiques comme par exemple les émollients ou adoucissants comme les huiles d'amande douce, de noyau d'abricot, les agents hydratants comme la glycérine, les agents apaisants comme l'$\alpha$-bisabolol, l'allantoïne, aloes vera ; les vitamines et leurs dérivés, les acides gras essentiels, les agents répulsifs contre les insectes, les propulseurs, les peptisants, des charges, des co-solvants, des filtres UV autres que ceux de formule (1), des stabilisants ou conservateurs, des colorants, des nacres, des paillettes et leurs mélanges.

9. Composition selon l'une quelconque des revendications 1 à 8, se présentant sous forme d'eau fraîche, d'eau de toilette, d'eau de parfum, de lotion après-rasage, d'eau de soin, d'huile de soin siliconée ou hydrosiliconée ; de lotion bi-phasique parfumée.

10. Composition selon l'une quelconque des revendications 1 à **9,** conditionnée sous forme de flacon ; conditionnée sous forme pressurisée dans un spray, un aérosol, un dispositifs piézoélectrique.

11. Procédé de parfumage des matières kératiniques humaines ou d'un vêtement, comprenant l'application sur lesdites matières kératiniques ou ledit vêtement de la composition telle que définie dans les revendications 1 à **10.**

12. Utilisation d'au moins une silicone benzotriazole de formule (I) tel que défini dans l'une quelconque des revendications précédentes, comme agent stabilisant des propriétés organoleptiques d'une composition cosmétique parfumante colorée vis-à-vis des agressions extérieures notamment de la lumière ou des différences de température en particulier la couleur et/ou l'odeur de ladite composition ; ladite composition ne contenant pas de composé $\beta,\beta'$-diphénylacrylate d'alkyle ni de composé $\alpha$-cyano-$\beta,\beta'$-diphénylacrylate d'alkyle.

**Patentansprüche**

1. Gefärbte Parfümierungszusammensetzung, umfassend in einem kosmetisch unbedenklichen Medium:

a) mindestens 2 Gew.-% einer Parfümierungssubstanz, bezogen auf das Gesamtgewicht der Zusammensetzung;
b) mindestens ein Benzotriazolsilikon der folgenden Formel (1):

$$O_{(3-a)/2}Si(R)_a\text{-}G \qquad (1)$$

worin:

- R für einen gegebenenfalls halogenierten $C_1$-$C_{10}$-Alkylrest oder einen Phenylrest oder einen Trimethylsilyloxyrest steht,
- a für eine ganze Zahl steht, die zwischen 0 und 3 inklusive ausgewählt ist,
- und das Symbol G für einen einwertigen Rest steht, der direkt an ein Siliciumatom gebunden ist und der folgenden Formel (2) entspricht:

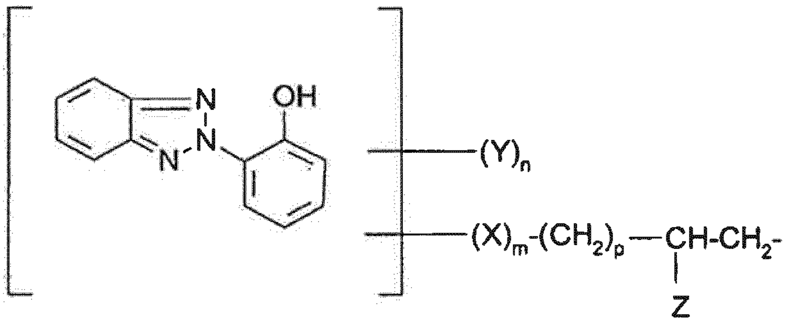

(2)

worin:

- die Variablen Y gleich oder verschieden sind und aus $C_1$-$C_8$-Alkylresten, Halogenen und $C_1$-$C_4$-Alkoxyresten ausgewählt sind, mit der Maßgabe, dass in diesem letzteren Fall zwei benachbarte Y an demselben aromatischen Kern zusammen eine Alkylidendioxygruppe bilden können, wobei die Alkylidengruppe 1 bis 2 Kohlenstoffatome enthält,
- X für O oder NH steht,
- Z für Wasserstoff oder einen $C_1$-$C_4$-Alkylrest steht,
- n für eine ganze Zahl zwischen 0 und 3 inklusive steht,
- m für 0 oder 1 steht,
- p für eine ganze Zahl zwischen 1 und 10 inklusive steht;

c) mindestens ein in dem Medium lösliches Farbmittel; wobei die Zusammensetzung keine β,β'-Diphenylacrylsäurealkylesterverbindung oder α-Cyano-β,β'-diphenylacrylsäurealkylesterverbindung enthält.

**2.** Zusammensetzung nach Anspruch 1, wobei das siliciumhaltige Benzotriazolderivat der Formel (I) aus den Verbindungen ausgewählt ist, die den folgenden Formeln (5) oder (6) entsprechen:

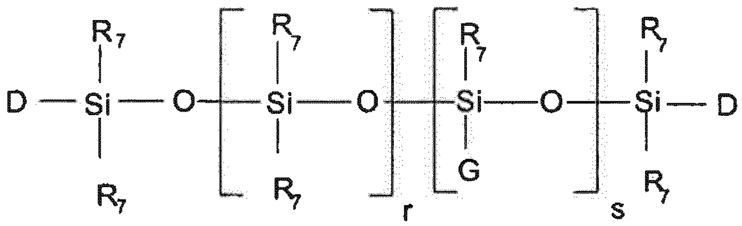

(5)

oder

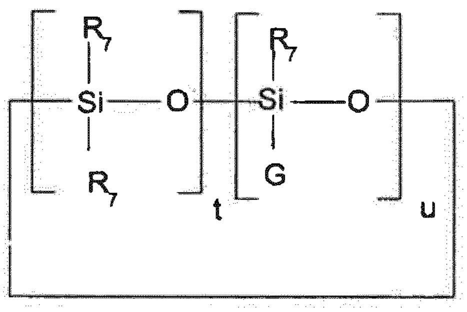

(6)

worin:

- die Variablen $R_7$ gleich oder verschieden sind und aus $C_1$-$C_{10}$-Alkyl-, Phenyl-, Trifluor-3,3,3-propyl-und Trimethylsilyloxyresten ausgewählt sind, wobei zahlenmäßig mindestens 80% der Reste $R_7$ für Methyl stehen,
- die Variablen D gleich oder verschieden sind und aus den Resten $R_7$ und dem Rest G ausgewählt sind,
- r für eine ganze Zahl zwischen 0 und 50 inklusive steht und s für eine ganze Zahl zwischen 0 und 20 inklusive steht und im Fall von s = 0 mindestens eines der beiden Symbole D für G steht,
- u für eine ganze Zahl zwischen 1 und 6 inklusive steht und t für eine ganze Zahl zwischen 0 und 10 inklusive steht, mit der Maßgabe, dass t + u größer gleich 3 ist,
- und das Symbol G der obigen Formel (2) entspricht.

3. Zusammensetzung nach Anspruch 2, wobei das siliciumhaltige Benzotriazolderivat der Formel (I) aus den Verbindungen ausgewählt ist, die der Formel (5) entsprechen.

4. Zusammensetzung nach Anspruch 2 oder 3, wobei das siliciumhaltige Benzotriazolderivat der Formel (I) aus den Verbindungen ausgewählt ist, die der Formel (5) entsprechen, für die die Reste D beide für $R_7$-Reste stehen.

5. Zusammensetzung nach einem der Ansprüche 2 bis 4, wobei das siliciumhaltige Benzotriazolderivat der Formel (5) mindestens eine und noch weiter bevorzugt alle der folgenden Eigenschaften aufweist:

- D steht für einen $R_7$-Rest,
- $R_7$ steht für Alkyl und noch weiter bevorzugt für Methyl,
- r liegt zwischen 0 und 15 inklusive; s liegt zwischen 1 und 10 inklusive,
- n ist nicht 0 und vorzugsweise gleich 1, und Y ist dann aus Methyl, tert.-Butyl oder $C_1$-$C_4$-Alkoxy ausgewählt,
- Z steht für Wasserstoff oder Methyl,
- m = 0 oder [m = 1 und X = O],
- p ist gleich 1.

6. Zusammensetzung nach einem der Ansprüche 1 bis 6, wobei die Verbindung der Formel (1) aus den Verbindungen ausgewählt ist, die der folgenden Formel (7) entsprechen:

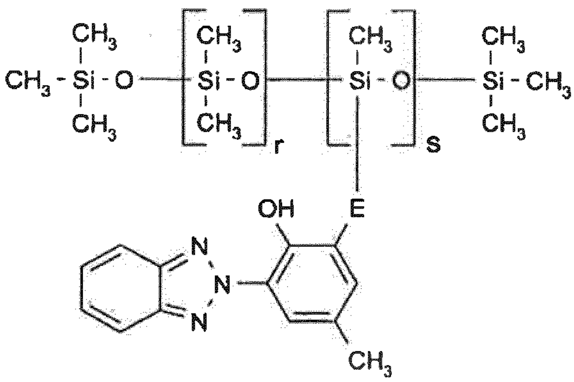

(7)

mit $0 \leq r \leq 10$,

$$1 \leq s \leq 10,$$

und wobei E für den folgenden zweiwertigen Rest steht:

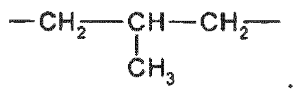

7. Zusammensetzung nach Anspruch 6, wobei es sich bei der Verbindung der Formel (7) um die Verbindung Drometrizole Trisiloxane handelt, die der folgenden Formel entspricht:

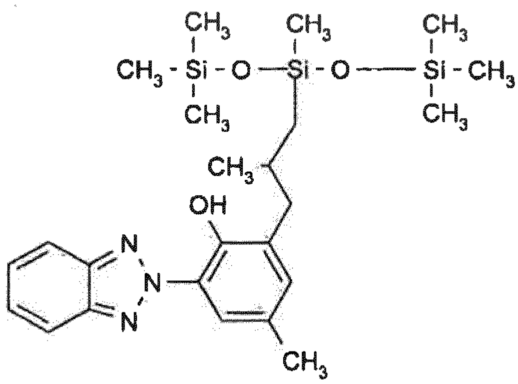

8. Zusammensetzung nach einem der Ansprüche 1 bis 7, außerdem umfassend mindestens ein Additiv, das aus Antioxidantien, Fettsubstanzen wie Ölen (pflanzlichen, mineralischen oder synthetischen Ölen wie Estern oder Perfluorethern), kosmetischen oder dermatologischen Wirkstoffen wie beispielsweise Emollientien oder zartmachenden Mitteln wie Süßmandelöl oder Aprikosenkernöl, Feuchtigkeitsmitteln wie Glycerin, Verdickungsmitteln wie α-Bisabolol, Allantoin oder Aloe vera; Vitaminen und deren Derivaten, essenziellen Fettsäuren, Schutzmitteln gegen Insekten, Treibmitteln, Peptisierungsmitteln, Füllstoffen, Cosolventien, UV-Schutzmitteln, die von denjenigen der Formel (1) verschieden sind, Stabilisatoren oder Konservierungsmitteln, Farbmitteln, Perlmutt, Glitzerplättchen und Mischungen davon ausgewählt ist.

9. Zusammensetzung nach einem der Ansprüche 1 bis 8, die in Form eines Eau fraîche, eines Eau de Toilette, eines Eau de Parfum, einer Aftershave-Lotion, eines Pflegewassers, eines gegebenenfalls Wasser enthaltenden Silikonpflegeöls oder einer parfümierten zweiphasigen Lotion vorliegt.

10. Zusammensetzung nach einem der Ansprüche 1 bis 9, die in Form einer Flasche oder in mit Druck beaufschlagter Form in einem Spray, einem Aerosol oder einer piezoelektrischen Vorrichtung abgepackt ist.

11. Verfahren zur Parfümierung von menschlichen Keratinmaterialien oder einem Kleidungsstück, bei dem man auf die Keratinmaterialien bzw. das Kleidungsstück die Zusammensetzung gemäß den Ansprüchen 1 bis 10 aufbringt.

12. Verwendung mindestens eines Benzotriazolsilikonderivats der Formel (I) gemäß einem der vorhergehenden Ansprüche als Mittel zur Stabilisierung der organoleptischen Eigenschaften einer gefärbten parfümierenden kosmetischen Zusammensetzung gegenüber Angriffen von außen, insbesondere Licht oder Temperaturdifferenzen, insbesondere der Farbe und/oder des Geruchs der Zusammensetzung; wobei die Zusammensetzung keine β,β'-Diphenylacrylsäurealkylesterverbindung oder α-Cyano-β,β'-diphenylacrylsäurealkylesterverbindung enthält.

## Claims

1. Coloured scenting composition comprising, in a cosmetically acceptable medium:

a) at least 2% by weight of a scenting substance, with respect to the total weight of the composition ;
b) at least one benzotriazole silicone of following formula (1):

$$O_{(3-a)/2}Si(R)_a\text{-}G \qquad (1)$$

in which:

- R represents an optionally halogenated $C_1$-$C_{10}$ alkyl radical or a phenyl radical or a trimethylsilyloxy radical,
- a is an integer chosen between 0 and 3 inclusive,
- and the G symbol denotes a monovalent radical bonded directly to a silicon atom which corresponds to the following formula (2):

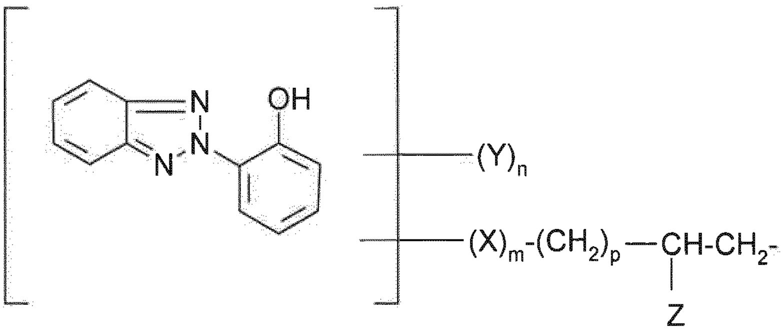

(2)

in which:

- Y, which are identical or different, are chosen from $C_1$-$C_8$ alkyl radicals, halogens and $C_1$-$C_4$ alkoxy radicals, it being understood that, in the latter case, two adjacent Y radicals on the same aromatic nucleus can together form an alkylidenedioxy group in which the alkylidene group comprises from 1 to 2 carbon atoms,
- X represents O or NH,
- Z represents hydrogen or a $C_1$-$C_4$ alkyl radical,
- n is an integer between 0 and 3 inclusive,
- m is 0 or 1,
- p represents an integer between 1 and 10 inclusive;

c) at least one dye which is soluble in the said medium; the said composition not comprising an alkyl β,β-diphenylacrylate or alkyl α-cyano-β,β-diphenylacrylate compound.

**2.** Composition according to Claim 1, where the benzotriazole silicon derivative of formula (1) is chosen from the compounds corresponding to the following formulae (5) and (6):

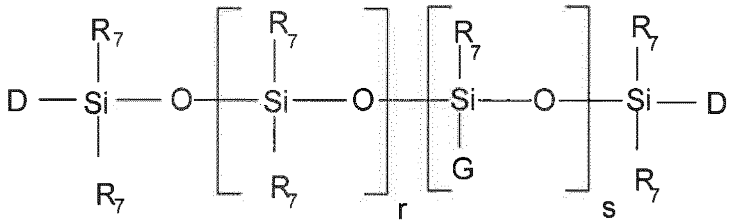

(5)

or

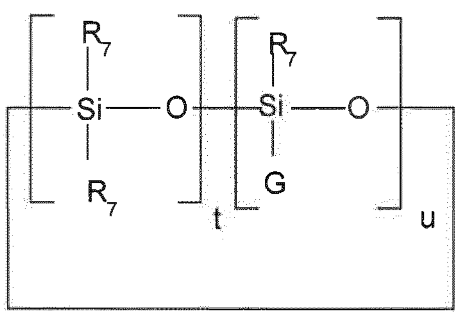

(6)

in which:

- $R_7$, which are identical or different, are chosen from $C_1$-$C_{10}$ alkyl, phenyl, 3,3,3-trifluoropropyl and trimethylsilyloxy radicals, at least 80% by number of the $R_7$ radicals being methyl,
- D, which are identical or different, are chosen from $R_7$ radicals and the G radical,
- r is an integer between 0 and 50 inclusive and s is an integer between 0 and 20 inclusive and, if s = 0, at least one of the two D symbols denotes G,
- u is an integer between 1 and 6 inclusive and t is an integer between 0 and 10 inclusive, it being understood that t + u is equal to or greater than 3,
- and the G symbol corresponds to the above formula (2).

**3.** Composition according to Claim 2, where the benzotriazole silicon derivative of formula (1) is chosen from the compounds corresponding to the formula (5).

**4.** Composition according to Claim 2 or 3, where the benzotriazole silicon derivative of formula (1) is chosen from the compounds corresponding to the formula (5) in which the D radicals are both $R_7$ radicals.

**5.** Composition according to any one of Claims 2 to 4, where the benzotriazole silicon derivative of formula (5) exhibits at least one and more preferably still all of the following characteristics:

- D is an $R_7$ radical,
- $R_7$ is alkyl and more preferably still is methyl,
- r is between 0 and 15 inclusive; s is between 1 and 10 inclusive,
- n is non-zero and preferably equal to 1 and Y is then chosen from methyl, tert-butyl or $C_1$-$C_4$ alkoxy,
- Z is hydrogen or methyl,
- m = 0 or [m = 1 and X = O],
- p is equal to 1.

**6.** Composition according to any one of Claims 1 to 6, where the compound of formula (1) is chosen from the compounds corresponding to the following formula (7):

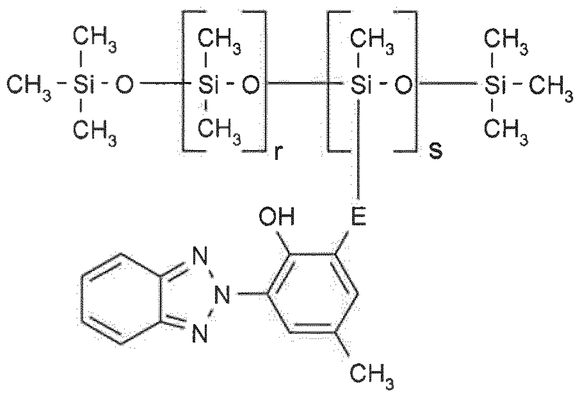

(7)

with $0 \leq r \leq 10$,

$$1 \leq s \leq 10,$$

and where E represents the divalent radical:

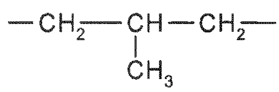

**7.** Composition according to Claim 6, where the compound of formula (7) is the compound Drometrizole Trisiloxane corresponding to the following formula:

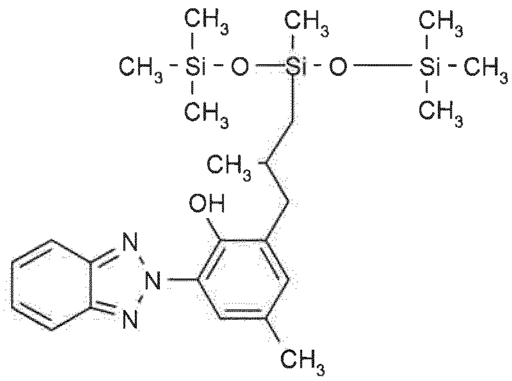

8. Composition according to any one of Claims 1 to 7, additionally comprising at least one additive chosen from antioxidants, fatty substances, such as oils (vegetable, mineral or synthetic oils, such as esters, or perfluoroethers), cosmetic or dermatological active principles, such as, for example, emollients or softening agents, such as sweet almond oil or apricot kernel oil, moisturizing agents, such as glycerol, soothing agents, such as $\alpha$-bisabolol, allantoin or aloe vera; vitamins and their derivatives, essential fatty acids, insect repellents, propellants, peptizing agents, fillers, cosolvents, UV screening agents other than those of formula (1), stabilizing agents or preservatives, dyes, pearlescent agents, glitter and their mixtures.

9. Composition according to any one of Claims 1 to 8, which is provided in the form of an eau fraîche, eau de toilette, eau de parfum, aftershave lotion, care water, silicone-based or hydrosilicone-based care oil or scented two-phase lotion.

10. Composition according to any one of Claims 1 to 9, packaged in the form of a bottle or packaged in pressurized form in a pump-action spray, an aerosol or a piezoelectric device.

11. Method for scenting human keratinous substances or an item of clothing, comprising the application, to the said keratinous substances or the the said item of clothing, of the composition as defined in Claims 1 to 10.

12. Use of at least one benzotriazole silicone of formula (I) as defined in any one of the preceding claims as agent which stabilizes the organoleptic properties of a coloured scenting cosmetic composition with regard to external attacks, in particular light or temperature differences, especially the colour and/or the odour of the said composition, the said composition not comprising an alkyl $\beta,\beta$-diphenylacrylate compound or an alkyl $\alpha$-cyano-$\beta,\beta$-diphenylacrylate compound.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 1897592 A
- WO 2005042828 A
- WO 0025370 A
- WO 09059872 A
- FR 2923386
- EP 1994921 A
- FR 2916347
- FR 2916348
- FR 2916349
- EP 0392883 A
- EP 0660701 A
- EP 0708108 A
- EP 0711778 A
- EP 711779 A
- US 3220972 A
- US 3697473 A
- US 4340709 A
- US 4316033 A
- US 4328346 A
- EP 0742003 A
- WO 06013413 A
- WO 2007068371 A
- WO 2008155059 A
- US 4077441 A
- US 4850517 A
- EP 1897592 A1


### Littérature non-brevet citée dans la description


- **S. ARCTANDER.** *Perfume and Flavor Chemicals,* 1969
- **S. ARCTANDER.** *Perfume and Flavor Materials of Natural Origin*
- Flavor and Fragrance Materials. Allured Publishing Co, 1991